(19) Europäisches Patentamt
European Patent Office
Office européen des brevets

(11) Publication number: **0 061 157**
**A1**

(12) **EUROPEAN PATENT APPLICATION**

(21) Application number: 82102231.6

(22) Date of filing: 18.03.82

(51) Int. Cl.$^3$: C 07 C 103/82
A 61 K 31/165

(30) Priority: 19.03.81 JP 38706/81
21.09.81 JP 147746/81

(43) Date of publication of application:
29.09.82 Bulletin 82/39

(84) Designated Contracting States:
BE CH FR GB IT LI NL SE

(71) Applicant: Hokuriku Pharmaceutical Co.,Ltd
1-Chome, 3-14 Tatekawacho Katsuyamashi
Fukui(JP)

(72) Inventor: Koshinaka, Eiichi
2-6-3 Asahicho Katsuyamashi
Fukui(JP)

(72) Inventor: Ogawa, Nobuo
2-6-5 Asahicho Katsuyamashi
Fukui(JP)

(72) Inventor: Kurata, Sakae
1-6-56 Asahicho Katsuyamashi
Fukui(JP)

(72) Inventor: Yamagishi, Kagari
2-5-44 Asahicho Katsuyamashi
Fukui(JP)

(72) Inventor: Kato, Hideo, Dr.
1-11-27 Motomachi Katsuyamashi
Fukui(JP)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) **2-Benzoylamino substituted benzylamine derivative and a process for preparation thereof.**

(57) 2-benzoylamino-substituted benzylamine derivatives represented by formula:

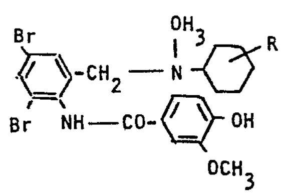

wherein R is a hydrogen atom or a hydroxyl group, and a pharmaceutically acceptable addition salt thereof are provided. These compounds are excellent expectorants having no toxicity.

EP 0 061 157 A1

2-BENZOYLAMINO-SUBSTITUTED BENZYLAMINE DERIVATIVE AND A PROCESS FOR PREPARATION THEREOF

## FIELD OF THE INVENTION

This invention relates to a new 2-benzoylamino-substituted benzylamine derivative and a pharmaceutically acceptable acid addition salt thereof which exhibits a potent expectorant activity and to a method for the preparation thereof. More particularly, this invention relates to a new 2-benzoylamino-substituted benzylamine derivative represented by formula (I):

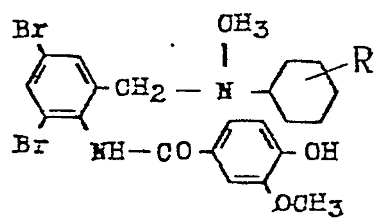

wherein R is a hydrogen atom and a hydroxyl group and a pharmaceutically acceptable acid addition salt thereof as well as to a method for the preparation thereof.

## DEVELOPMENT OF THE INVENTION

An expectorant which is heretofore known has two activities: one is to discharge sputum with cough by increasing tracheal secretion, fluidizing sputum adhered to respiratory tract and facilitating its separation from the wall of respiratory tract (a bronchoexcitosecretory action); another is to facilitate discharge by physicochemically reducing a viscosity of sputum (trachel mucous dissolution action).

Bromhexine (generic name, Merck Index, 9th Edition, 1389) represented by formula (II):

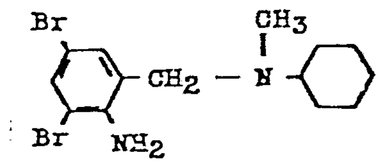

is known as a medicine which has both activities above and has been widely provided for clinical use. For purpose of improving an expectorant action, N-acylderivatives of bromhexine were prepared recently (Japanese Patent Application (OPI) 16640/76 corresponding to German Patent 2,440,596, British Patent 1,432,904, French Patent 2,259,595, Spanish Patent 418,443, hereafter the same) and brovanexine (generic name) of formula (III):

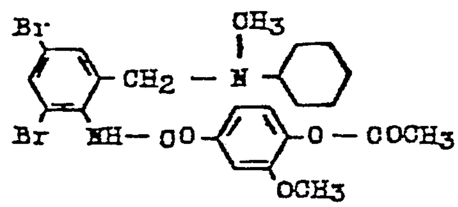

was recognized to be a good expectorant in screening of these derivatives and has been clinically used.

However, brovanexine of formula (III) shows no improvement in an expectorant activity as compared to bromhexine of formula (II) but shows a slight improvement in toxicity.

As a result of extensive investigations on new compounds having a potent expectorant activity, it has been found that compounds of formula (I) have extremely weak toxicity and possess a potent expectorant activity by approximately 10 times or more when compared to those of formulae (II) and (III), and thus this invention has been accomplished.

## SUMMARY OF THE INVENTION

The foregoing problems in the prior art have been solved by providing a novel 2-benzoylamino-substituted benzylamine derivative of formula (I) and a process for preparing the same.

## PREFERRED EMBODIMENTS OF THE INVENTION

Specific examples of compounds represented by formula (I) include:

2-(4-Hydroxy-3-methoxybenzoylamino)-3,5-dibromo-N-(cis-3-hydroxycyclohexyl)-N-methyl-benzylamine

2-(4-Hydroxy-3-methoxybenzoylamino)-3,5-dibromo-N-(trans-3-hydroxycyclohexyl)-N-methylbenzylamine

2-(4-Hydroxy-3-methoxybenzoylamino)-3, 5-dibromo-N-(trans-4-hydroxycyclohexyl)-methylbenzylamine

2-(4-Hydroxy-3-methoxybenzoylamino)-3, 5-dibromo-N-cyclohexyl-N-methylbenzylamine

Typical examples of pharmaceutically acceptable acid addition salts of the compound represented by formula (I) include salts of inorganic acids such as hydrochloric acid, sulfuric acid, nitric acid, phosphoric acid, hydrobromic acid, hydroiodic acid and the like, and organic acids such as acetic acid, maleic acid, fumaric acid, citric acid, oxalic acid, tartaric acid and the like.

In this invention, the new 2-benzoylamino-substituted benzylamine derivatives represented by formula (I) can be prepared as follows:

According to one embodiment of the process for preparing the compound in accordance with this invention, the compound represented by formula (I) can be prepared by hydrolyzing, in an aqueous medium, an ester compound of formula (IV)

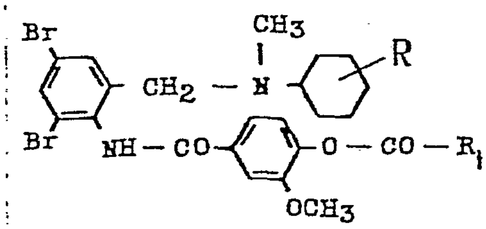

wherein, R is as defined above and $R_1$, is a lower alkyl group, preferably having 1 to 4 carbon atoms or a lower alkoxy group, preferably having 1 to 4 carbon atoms.

In term "aqueous medium" used in this invention refers to water or a polar solvent containing water. A preferred aqueous medium is a polar solvent (e.g. methanol, ethanol, etc) containing about 10 to about 50% (by volume) of water. Typical examples of aqueous media which are preferably employed in this invention are methanol or ethanol containing about 10 to about 50% (by volume) of water.

A particularly preferred embodyment of the process of this invention comprises hydrolyzing the ester compound represented by formula (IV) in an aqueous medium containing sodium hydroxide or potassium hydroxide.

The reaction is generally performed at temperatures of from about $0^oC$ to about $100^oC$ (preferably $0^o$ to $40^oC$, more preferably, room temperature (about $20^oC$) for about

10 minutes to about 16 hours (preferably 10 mins. to 2 hrs.). Unless otherwise indicated, the reaction is effected under normal pressure.

Starting materials represented by formula (IV) in which $R_1$ is a hydrogen atom and $R_1$ is a lower alkyl group are known as is described in, e.g., Japanese Patent Application (OPI) 16640/76. Other compounds of formula (IV) in which $R_1$ is a hydrogen atom and $R_1$ is a lower alkoxy group and in which R is a hydroxy group and $R_1$ is a lower alkyl group or a lower alkoxy group are all new compounds.

According to another embodiment of the process for preparing the compounds in accordance with this invention, the compounds represented by formula (I) can be prepared by reacting 2-amino-substituted benzylamine derivatives of formula (V).

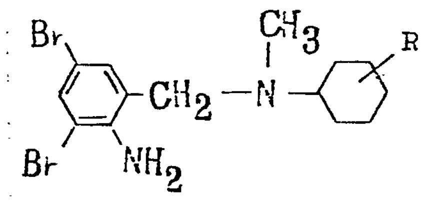

wherein R is as defined above, with vanillic acid of formula (VI)

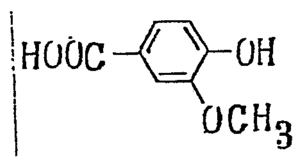

in the presence of a condensing agent.

The reaction is conducted in an organic solvent such as acetone, dioxane, acetonitrile, chloroform, methylenedichloride, tetrahydrofuran and the like.

Condensing agents which are employed in this invention

are agents for effecting condensation by removing water that are widely used in the art of peptide synthesis for purpose of forming an amide bond.

Representatives of such condensing agents are N, N'-dicyclohexyl-carbodiimide, N-cyclohexyl-N'-morphorino-ethyl-carbodiimide, N-cyclohexyl-N'-(4-diethyl-amino-cyclo-hexyl) carbodiimide and the like.

The condensation is carried out at temperatures of from 0°C to under reflux of a solvent used (preferably 10 to 40°C, more preferably room temperature - about 20°C) for 10 mins. to 5 hrs. (preferably 20 mins. to 1 hr.). The condensing agents, condensation procedures, etc, are described in, e.g. Fieser & Fieser, Reagents for Organic Synthesis, page 231 - 233 (1967), John Wiley & Sons, Inc. in detail.

Starting materials of formula (V) in the second embodiment of this invention, said formula (V) are all known and can be prepared in a manner as described in Japanese Patent Publications 5533/70 (corresponding to U.S. Patent 3,536,713), 20096/70 (corresponding to German Patent 1,593,-579), 20889/70 (corresponding to British Patent 1,178.034), 39809/70 (corresponding to French Patent 1,522,709), etc.

The thus prepared new 2-benzoylamino-substituted benzylamine derivatives of formula (I) and pharmaceutically acceptable acid addition salts thereof exhibit excellent expectorant activity and are extremely useful for medicine.

The compounds of this invention exhibit strong expectorant activity with minimized side effects. The high order of these activities of the active agent of this invention is evidenced by test in lower animals, representative of which are reported herein. The compound of this invention can be administered per os, e.g., in the form of pills or tables, in which it may be present together with the usual pharmaceutical carriers, conventionally by compounding the compounds of this invention together with a customary carrier or adjuvant, such as talc, magnesium stearate, starch, lactose, gelatin, any of numerous gums, and the like. Thus, in their most advantageous form, the compositions of this invention will contain a non-toxic pharmaceutical carrier in addition to the active ingredient of this invention. Exemplary solid carriers are lactose, magnesium stearate, calcium stearate, starch, terra alba, dicalcium acacia, or the like. Representative liquid carrers are peanut oil, sesame oil, olive oil, water, or the like. The active agents of this invention can be conveniently administered in such compositions containing active ingredient so as to eventually be within the dosage range illustrated hereafter. Thus, a wide variety of pharmaceutical forms suitable for many modes of adminstration and dosages may be employed. For oral administration, the active ingredient and pharmaceutical forms suitable for many modes of administration and dosages may be

employed. For oral administration, the active ingre-dient and pharmaceutical carrier may, for example, take the form of a granule, pill, tablet, lozenge, elixir, syrup, or other liquid suspension or emulsion, whereas, for parenteral administration, the composition may be in the form of a sterile solution or suppository.

The method of using the compounds of this invention comprises internally or externally administering the compounds of this invention, preferably orally or parenterally and preferably admixed with the pharmaceutical carrier, for example, in the form of any of the above compositions, or filled into a capsule, to alleviate conditions to be treated and symptoms thereof in a living animal body. Illustratively, it may be used in an amount of about 1 to about 100 mg. per unit dose, preferably 3 to 30 mg. for an oral dose, while parenteral dosages are usually less and ordinarily about one-half of the oral dose. The unit dose is preferably given a suitable number of times daily, typically three times. The daily dose may vary depending upon the number of times given. Naturally, a suitable clinical dose must be adjusted in accordance with the condition, age, and weight of the patient, and it goes without saying that the enhanced activities of the compounds of this invention, together with their reduced side effects, also make it suitable for wide variations, and the invention therefore should not be limited

by the exact ranges stated. The exact dosage, both unit dosage and daily dosage, will of course have to be determined according to established medical principles.

Expectorant activity is generally evaluated by two effects: one is an effect on acceleration of tracheal secretion and another is an effect on dissolution of mucus on the tracheal tract. The former is determined by examining an effect on the secretory activity of the tracheal secretory cells and the latter by examining an effect on the dissolution of acid glycoprotein in submucosal glandular cells of the trachea. As reference drugs, two compounds structurally similar to the compound of this invention were used.

(Compounds Tested)

A: Compound of This Invention (Example 7)

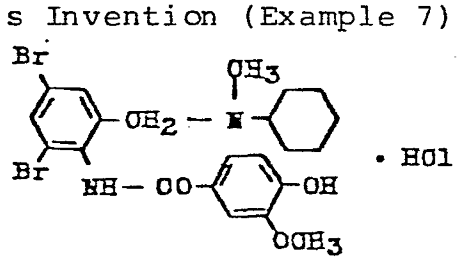

2-(4-Hydroxy-3-methoxybenzoylamino)-3, 5-dibromo-N-cyclohexyl-N-methylbenzylamine hydrochloride

B: Reference Drug 1 (brovanexine hydrochloride):

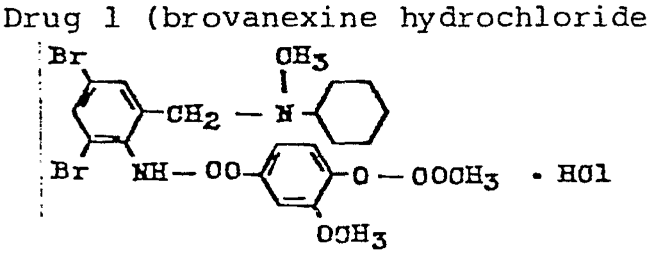

2-(4-Acetoxy-3-methoxybenzoylamino)-3.5-dibromo-N-cyclohexyl-N-methylbenzylamine hydrochloride

C: Reference Drug 2 (bromhexine hydrochloride):

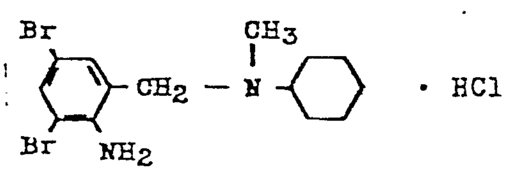

2-Amino-3, 5-dibromo-N-cyclohexyl-N-methylbenzylamine hydrochloride

(Experimental Method)

Experiments were performed following the method of Yanaura et al. (Nippon Yakurigaku Zasshi, 77, 559 - 568 (1981)).

Male mongrel dogs weighing 10 to 12 kg were anesthetized with sodium pentobarbital (30 mg/kg, i.v.). The trachea was quickly excised about 10 cm long and rapidly transfered to a Hanks' solution (95% $O_2$ - 5% $CO_2$, 37 $\pm$ $1^o$ ). Connective tissues around were removed and the trachea was cut into strips about 2 cm long. The tracheal strips were preincubated for 30 minutes in Hanks' solution, and then drugs were added to the solution and the mixtures were further incubated for 30 minutes. The tracheal strips were then fixed with a 10% formaldehyde isotonic solution followed by dehydrating in a series of graded ethanol concentrations and paraffinembedding in a conventional manner. Sections 4 μm thick were cut with a Jung type microtome and stained with alcian blue (pH 2.5) - periodic acid Schiff (AB (pH 2.5)/PAS). The specimens were studied by the optical microscopic observation with the effects of drugs on the secretory activities of the tracheal secretory cells and on the dissolution of acid glycoprotein in submucosal glandular cells of the tracheal.

1. Effect on the secretory activity of the tracheal secretory cells.

A thickness between basement membrance and cartilage

(thickness of endepidermis; wall is referred to as "W"), and acinar inner diameter of submucosal gland ($A^I$) were measured. Then, acinar inner diameter of submucosal gland to wall ratio ($A^IWR$) was calculated. Increase of $A^IWR$ reflects enhancement of the secretory activity in submucosal gland. If test drug has a significant increase in $A^IWR$, it has a respiratory tract fluid (RTF) - increasing effect.

Results are shown in Table 1.

Table I Effects on the secretory activity of the tracheal secretory cells.

| Compound drugs | Acinar Inner Diameter of Submucosal Gland to Wall Ratio ($A^IWR$) | | |
|---|---|---|---|
| | $1 \times 10^{-7}M$ | $1 \times 10^{-6}M$ | $1 \times 10^{-5}M$ |
| Compound of This invention | $6.1 \times 10^{-2}$* | $7.1 \times 10^{-2}$** | $7.3 \times 10^{-2}$** |
| Reference Drug 1 | $5.3 \times 10^{-2}$ | $6.2 \times 10^{-2}$* | $6.5 \times 10^{-2}$** |
| Reference Drug 2 | $5.2 \times 10^{-2}$ | $5.6 \times 10^{-2}$ | $6.2 \times 10^{-2}$* |
| Control | $5.0 \times 10^{-2}$ | " | " |

$A^IWR = A^I/W$

Significant Difference from Control:

* $P < 0.05$

** $P < 0.01$

It is clear that the compound of this invention significantly increased $A^IWR$ even at a lower dose of $1 \times 10^{-7}M$ and had a powerful enhancement of RTF secretory activity. In more detail, the compound of this invention showed a significant increase in $A^IWR$ at a dose of $1 \times 10^{-7}M$ whereas

neither reference drug 1 nor reference drug 2 showed any significant increase in $A^IWR$ as compared to control. Only reference drug 1 showed a significant increase in $A^IWR$ at a dose of 1 x $10^{-6}$M. In other words, the enhancement of the compound of this invention in $A^IWR$ was about 10 times as strong as that of reference drugs 1 and 2.

Based on the data in Table 1, a dose required for increasing $A^IWR$ of each of the compounds tested by 25% ($ED_{25}$) was calculated.

Results are shown in Table II in which $ED_{50}$ is expressed by an increasing rate (%) in $A^IWR$ of control to that of each of the compounds at a given dose.

Table II

| Compound | Increasing Rate in $A^IWR$ (%) 1 x $10^{-7}$M | 1 x $10^{-6}$M | 1 x $10^{-5}$M |
|---|---|---|---|
| Compound of this invention | 22* | 42** | 46** |
| Reference Drug 1 | 6 | 24* | 30** |
| Reference Drug 2 | 4 | 12 | 24* |
| Control | - | - | - |

2. Effect on the Dissolution of Acid Glycoprotein in Submucosal Glandular Cells of the Trachea.

As a tracheal mucus dissolution activity, an effect on the dissolution of acid glycoprotein in submucosal glandular cells was observed.

In double staining with AB (pH 2.5)/PAS, submucosal glandular cells containing much acid glycoprotein were strongly stained blue because of the reaction with AB. When acid glycoprotein in glandular cells is dissolved and its acid groups were lost, it is stained with PAS instead of AB. Consequently, submucosal glandular cells turned to red from blue in color. The ratio of blue glandular cells to total glandular cells was observed, and the effect on the dissolution of acid glycoprotein was estimated by decrease in the ratio.

Results are shown in Table III.

Table III Effects on the dissolution of acid glycoprotein in submucosal glandular cells of the trachea.

| | Ratio of Glandular Cells Containing Acid Glycoprotein to Total Glandular Cells (%) | | |
|---|---|---|---|
| Compound | $1 \times 10^{-7}$ M | $1 \times 10^{-6}$ M | $1 \times 10^{-5}$ M |
| Compound of This Invention | 42.0** | 21.6** | 10.6** |
| Reference Drug 1 | 71.0 | 46.8** | 23.8** |
| Reference Drug 2 | 66.2 | 30.0** | 17.5** |
| Control | 72.4 | " | " |

Significant Difference from Control:

** $P < 0.01$

It was found that the compound of this invention had

a powerful mucolytic property since this compound markedly dissolved acid glycoprotein which was thought to be a main component responsible for mucus viscosity in the respiratory tract. This activity was 10 times stronger than that of reference drugs 1 and 2.

Based on the above data, a dose of each of the compounds tested required for decreasing acid glycoprotein by 50% ($ED_{50}$) was calculated. Further, a potency ratio was determined when the dose of reference drug 1 was made 1.

Results are shown in Table IV.

Table IV

| Compound | $ED_{50}$ | Potency Ratio |
|---|---|---|
| Compound of This Invention | $2.3 \times 10^{-7}M$ | 10.87 |
| Reference Drug 1 | $2.5 \times 10^{-6}M$ | 1.00 |
| Reference Drug 2 | $1.3 \times 10^{-6}M$ | 1.92 |

It is clearly seen from the results above that the compound of this invention provided potent mucolytic property as compared to reference drugs 1 and 2.

(Acute toxicity test)

Male mice of ddy-strain weighing 20 to 25 g were used. Drugs were administered orally and intraperitoneally to 10 mice in each group. $LD_{50}$ was calculated by the Litchfield-Wilcoxon method from dead animals within 7 days.

Results are shown in Table V.

Table V. Acute Toxicity in Mice

| Compound | $LD_{50}$ (mg/kg) P.O. | $LD_{50}$ (mg/kg) I.P. |
|---|---|---|
| Compound of This Invention | > 12000* | > 4000* |
| Reference Drug 1 | > 12000* | > 4000* |
| Reference Drug 2 | 9000 | 2320 |

*This dose was the limit that mice could accept. Accordingly, it was impossible to determine $LD_{50}$.

This invention will be described in detail with reference to the examples below:

## Example 1

2-(4-Acetoxy-3-methoxybenzoyamino)-3, 5-dibromo-N-(cis-3-hydroxycyclohexyl)-N-methylbenzylamine hydrochloride:

A solution of 4-acetoxy-3-methoxybenzoylchloride (prepared by the reaction of 4.21g of 4-acetoxybenzoic acid with 2.15ml of thionyl chloride in conventional manner) in 20ml of chloroform is dropwise added to a solution of 6.55 g of 2-amino-3, 5-dibromo-N-(cis-3-hydroxycyclohexyl)-N-methyl-

benzylamine in 20ml of chloroform at room temperature. After completion of the addition, the mixture is heated under reflux for an hour. After cooling, the precipitate is filtered by suction to yield 9.56g of a colorless crystal. Recrystallization of the crude product from ethanol yields a colorless imbricate cristalline product having a m.p. of 247 - 249°C.

Elemental Analysis for $C_{24}H_{28}Br_2N_2O_6.HCl$:
Calcd. C, 46.44; H, 4.71; N. 4.51
Found: C, 46.21; H, 4.71; N, 4.25

## Example 2

2-(4-Acetoxy-3-methoxybenzoylamino)-3, 5-dibromo-N-(trans-3-hydroxycyclohexyl)-N-methylbenzylamine:

A solution of 4-acetoxy-3-methoxybenzoylchloride (prepared by the reaction of 2.65 g of 4-acetoxy-3-methoxybenzoic acid with 1.40 ml of thionyl chloride in a conventional manner) in 20 ml of chloroform is dropwise added to a solution of 6.55g of 2-amino-3, 5-dibromo-N-(cis-3-hydroxycyclohexyl)-N-methylbenzylamine in 20ml of chloroform at room temperature. After completion of the addition, the mixture is heated under reflux for an hour. after cooling, the reaction solution is rendered weakly alkaline with a saturated aqueous sodium bicarbonate solution and extracted with chloroform. After washing the chloroform layer with water and drying, the solvent is removed by distillation. The obtained

residue is washed with isopropylether to yield 5.26g of a colorless cristal. Recrystallization from ethyl acetate yields a colorless prism crystal having a m.p. of 166 - 167°C

Elemental Analysis for $C_{24}H_{28}Br_2N_2O_5$:

Calcd: C, 49.33; H, 4.83; N, 4.79

Found: C, 49.45; H, 5.08; N, 4.54

Example 3

2-(4-Acetoxy-3-methoxybenzoylamino)-3, 5-dibromo-N-(trans-4-hydroxycyclohexyl)-N-methylbenzylamine;

A solution of 4-acetoxy-3-methoxybenzoylchloride (prepared by the reaction of 9.64 g of 4-acetoxy-3-methoxybenzoic acid with 4.91 ml of thionyl chloride in a conventional manner) is dropwise added to a solution of 15.0g of 2-amino3, 5-dibromo-N-(trans-4-hydroxycyclohexyl)-N-methylbenzylamine in 50ml of chloroform at room temperature. After completion of the addition, the reactin mixture is heated under reflux for half an hour. after cooling, the reaction solution is rendered weakly alkaline with a saturated aqueous sodium bicarbonate solution and extracted with chloroform. After the chloroform layer is washed with water and dried, the solvent is removed by distillation to yield 28.6 g of a viscous brown liquid.

Example 4

2-(4-Hydroxy-3-methoxybenzoylamino)-3, 5-dibromo-N-(cis-3-hydroxycyclohexyl)-N-methylbenzylamine:

A 20% sodium hydroxide aqueous solution (7.7ml) is

added to a suspension of 8.00g of 2-(4-acetoxy-3-methoxybenzoyamino)-3, 5-dibromo-N-(cis-3-hydroxycyclohexyl)-N-methybenzylamine hydrochloride prepared as in Example 1 in 100ml of methanol. The mixture is stirred at room temperature for 10 minutes. After completion of the reaction, methanol is distilled off and the residue is acidified with a hydrochloric acid aqueous solution. The aqueous layer is then removed by decantation to obtain a gum residue. The aqueous layer is washed with ether. The gum residue is dissolved in a 10% methanol-chloroform solution and the aqueous layer washed with ether is added to the solution. The solution obtained is rendered alkaline with sodium bicarbonate and extracted with chloroform. After the chloroform layer is washed with water and dried, the solvent is distilled off. Recrystallization of the residue from an ethanol-water mixture yields 4.76g of a colorless prism crystal having a m.p. of 182-183 °C.

Elemental Analysis for $C_{22}H_{26}Br_2N_2O_4$:
Calcd: C, 48.73; H, 4.83; N, 5.17
Found: C, 48.53, H, 4.93; N, 4.91

Example 5

2-(4-Hydroxy-3-methoxybenzoylamino)-3, 5-dibromo-N-(trans-3-hydroxycyclohexyl)-N-methylbenzylamine

A 20% sodium hydroxide aqueous solution (3.5ml) is added to a suspension of 4.60g of 2-(4-acetoxy-3-methoxy-

 0061157

benzoylamino)-3, 5-dibromo-N-(trans-3-hydroxycyclohexyl)-N-methylbenzylamine prepared as in Example 2 in 40ml of methanol. The mixture is stirred at room temperature for 15 minutes. After completion of the reaction, methanol is distilled off and the residue is acidified with a hydrochloric acid aqueous solution. Thereafter, the aqueous layer is removed by decantation to obtain a gum residue. The gum residue is dissolved in a 10% methanol-chloroform mixture and the aqueous layer described above is added thereto. The solution obtained is rendered alkaline with sodium bicarbonate and extracted with chloroform. After the chloroform layer is washed with water and dried, the solvent is distilled off. Recrystallization of the residue from a ethanol-water mixture yields 3.15g of a colorless needle crystal having a m.p. of 215.5 - 216.5$^{o}$C.

Elemental Analysis for $C_{22}H_{26}Br_2N_2O_4$:

Calcd: C, 48.73; H, 4.83; N, 5.17

Found: C, 48,87; H, 4.85; N, 4.94

Example 6

2-(4-Hydroxy-3-methoxybenzoylamino)-3, 5-dibromo-N-(trans-4-hydrocyclohexyl)-N-methylbenzylamine:

To a solution of 20.0 g of 2-(4-acetoxy-3-methoxybenzoylamino)-3, 5-dibromo-N-(trans-4-hydroxycyclohexyl)-N-methybenzylamine in 200ml of methanol, 13ml of a 20% sodium

hydroxide aqueous solution is added and the mixture is stirred at room temperature for 10 minutes. After completion of the reaction, methanol is distilled off and the residue is acidified with a hydrochloride aqueous solution. After washing with ether; the solution is rendered alkaline with potassium carbonate and a precipitated crystal is filtered. Recrystallization from a methanol-water mixture yields 9.61g of a colorless prism crystal having a m.p. of 194 - 195°C.

Elemental Analysis for $C_{22}H_{26}Br_2N_2O_4$.HCl.1/2 $H_2O$:

Calcd: C, 44.98; H, 4.80; N, 4.77

Found: C, 45.09; H, 4.65; N, 4.67

Example 7

2-(4-Hydroxy-3-methoxybenzoylamino)-3, 5-dibromo-N-cyclohexyl-N-methylbenzylamine

To a suspension of 160g of 2-(4-acetoxy-3-methoxybenzoylamino)-3, 5-dibromo-N-cyclohexyl-N-methylbenzylamine hydrochloride in 160ml of methanol, 160ml of a 20% sodium hydroxide aqueous solution is added and the mixture is stirred at room temperature for four hours. After completion of the reaction, methanol is distilled off under reduced pressure. To the residue, 480ml of chloroform is added. Then, the solution is rendered weakly alkaline with a 20% potassium carbonate solution and then extracted with chloroform. After the chloroform layer is washed and dried, the solvent is distilled off. Recrystallization of the residue from methanol yields 128g of a colorless prism crystal having a m.p. of 199 - 200°C.

Elemental Analysis for $C_{22}H_{26}Br_2N_2O_3$:

Calcd: C, 50.21; H, 4.98; N, 5.32

Found: C, 50.08; H, 4.94; N, 5.23

The thus obtained base (10.0g) is dissolved in 150ml of chloroform and concentrated hydrochloric acid is added thereto by stirring and cooling. A precipitated crystal is taken up by filteration. After washing the crystal with water, 16ml of hot methanol is added thereto and the mixture is stirred for 5 minutes followed by filtering a precipitate. Recrystallization from ethanol yields 7.51g of the colorless needle crystalline hydrochloride salt having a m.p. of 242 - 243°C (decomposed).

Elemental Analysis for $C_{22}H_{26}Br_2N_2O_3.HCl$:

Calcd: C, 46.96; H, 4.84; N, 4.98

Found: C, 46.79; H, 4.79; N, 4.74

<u>Example 8</u>

2-(4-Ethoxycarbonyloxy-3-methoxybenzoylamino)-3, 5-dibromo-N-cyclohexyl-N-methylbenzylamine

A solution of 4.80g of 4-ethoxycarbonyloxy-3-methoxybenzoylchloride (prepared by the reaction of 4.80g of 4-ethoxycarbonyloxy-3-methoxybenzoic acid with 5 ml of thionylchloride in a conventional manner) in 10 ml of chloroform is dropwise added to a solution of 6.40g of 2-amino-3, 5-dibromo-N-cyclohexyl-N-methylbenzylamine in 30ml of chloroform with heating under reflux. After completion of the dropwise addition, heating under reflux is continued for an hour.After cooling, the solution is rendered alkaline with a potassium carbonate aqueous solution and then extracted with chloroform. After the chloroform layer is washed with water and dried, the solvent is distilled off. Recrystallization of the residue from ethanol yields 8.87g of a colorless needle crystal having a m.p. of 148 - 150$^{o}$C.

Elemental Analysis for $C_{25}H_{30}Br_2N_2O_5$:
Calcd: C, 50.19; H, 5.05; N, 4.68
Found: C, 50.16; H, 4.98; N, 4.43

Example 9

2-(4-Hydroxy-3-methoxybenzoylamino)-3, 5-dibromo-N-cyclohexyl-N-methylbenzylamine hydrochloride salt:

To a suspension of 5.00g of 2-(4-ethoxycarbonyloxy-3-methoxybenzoylamino)-3, 5-dibromo-N-cyclohexyl-N-methylbenzylamine in 30ml of methanol, 3.0ml of a 20% sodium hydroxide aqueous solution is added and the mixture is stirred at room temperature for an hour. The reaction solution is neutralized with concentrated hydrochloric acid under cooling and the solvent is then distilled off. To the residue obtained chloroform is added and the mixture is rendered weakly alkaline with a sodium bicarbonate aqueous solution and then extracted with chloroform. After the chloroform layer is

washed with water and dried, the solvent is distilled off. To the residue obtained, a 10% hydrochloride aqueous solution is added and a precipitated crystal is filtered. Recrystallization of the precipitate from ethanol yields 4.48g of a colorless needle crystal having a m.p. of 242 - 243°C (decomposed).

A sample is identified to be the crystal obtained in Example 7 with IR spectrum.

Example 10

2-(4-Hydroxy-3-methoxybenzoylamino)-3, 5-dibromoNcyclohexyl-Nmethylbenzylamine hydrochloride salt:

Dicyclohexylcarbodiimide (0.49g) is added to a suspension of 0.75g of 2-amino-3, 5-dibromo-N-cyclohexyl-N-methylbenzylamine and 0.50g of vanillic acid in 10ml of chloroform. The mixture is stirred at room temperature for 20 minutes. After completion of the reaction, the precipitate is filtered off. The filtrate is washed with sodium bicarbonate and then concentrate under reduced pressure. The residue is acidified with dilute hydrochloric acid and the precipitate is filtered. Recrystallization of the precipitate from ethanol yields 0.37g of a colorless needle crystal having a m.p. of 242 - 243°C (decomposed).

A sample is identified to be the crystal obtained in Example 7 with IR spectrum.

-v. Pechmann-
Lehrens-Goetz
...erstraße 2
München 90



What is claimed:

1. A 2-benzoylamino-substituted benzylamine derivative represented by formula:

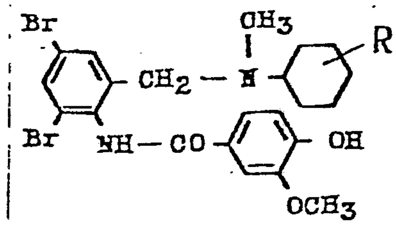

wherein R is a hydrogen atom or a hydroxyl group, and a pharmaceutically acceptable acid addition salt thereof.

2. The 2-benzoylamino-substituted benzylamine derivative of claim 1 wherein said hydroxyl group is on the 3 position of the cyclohexane nucleus.

3. The 2-benzoylamino-substituted benzylamine derivative of claim 1 wherein said hydroxyl group is on the 4 position of the cyclohexane nucleus.

4. 2-(4-Hydroxy-3-methoxybenzoylamino)3, 5dibromoN-cyclohexyl-N-methylbenzylamine.

5. A process for preparation of a 2-benzoylamino-substituted benzylamine derivative represented by formula:

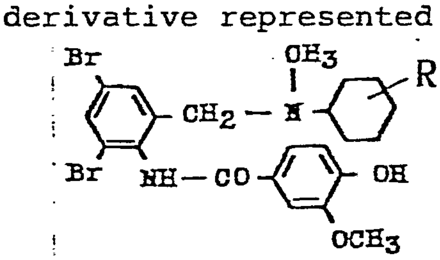

wherein R is a hydrogen atom or a hydroxyl group, and a pharmaceutically acceptable acid addition salt thereof which comprises hydrolyzing in an aqueous medium an ester compound of formula:

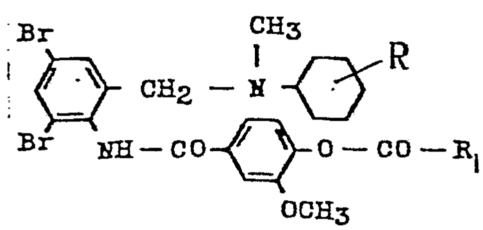

wherein R is as defined above and $R_1$ is a lower alkyl group or a lower alkoxy group.

6. A process for the preparation of a 2-benzoylamino-substituted benzylamine derivative represented by formula:

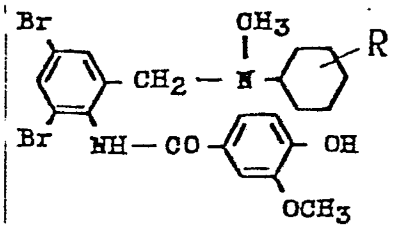

wherein R is a hydrogen atom or a hydroxyl group, and a pharmaceutically acceptable acid addition salt thereof, which comprises reacting 2-amino-substituted benzylamine derivative of formula:

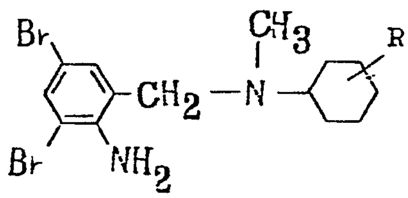

wherein R is as defined above, with vanillic acid of formula:

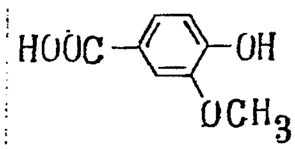

in the presence of a condensing agent.

European Patent Office

# EUROPEAN SEARCH REPORT

0061157

Application number

EP 82 10 2231

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | DE-A-2 440 546 (J. URIACH)<br>* pages 1-4; examples 23-32 *<br>----- | 1,4-6 | C 07 C 103/82<br>A 61 K 31/165 |
| | | | **TECHNICAL FIELDS SEARCHED (Int. Cl. 3)** |
| | | | C 07 C 103/00<br>A 61 K 31/00 |

The present search report has been drawn up for all claims

| Place of search | Date of completion of the search | Examiner |
|---|---|---|
| THE HAGUE | 10-06-1982 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82